(19) Europäisches Patentamt
European Patent Office
Office européen des brevets

(11) **EP 3 719 000 A1**

(12) **EUROPEAN PATENT APPLICATION**
published in accordance with Art. 153(4) EPC


(43) Date of publication:
**07.10.2020 Bulletin 2020/41**

(21) Application number: **18884471.6**

(22) Date of filing: **01.11.2018**

(51) Int Cl.:
*C07C 67/303* (2006.01) *C07C 69/75* (2006.01)
*B01J 23/46* (2006.01) *C08K 5/00* (2006.01)
*C08K 5/101* (2006.01) *C08L 101/00* (2006.01)

(86) International application number:
**PCT/KR2018/013196**

(87) International publication number:
**WO 2019/107772 (06.06.2019 Gazette 2019/23)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **29.11.2017 KR 20170161951**



(71) Applicant: **Hanwha Solutions Corporation**
**Jung-gu**
**Seoul 04541 (KR)**

(72) Inventors:
- **LEE, Hye Won**
  **Daejeon 305-720 (KR)**
- **KIM, Hyo Suk**
  **Daejeon 34023 (KR)**
- **JUNG, Ki Taeg**
  **Daejeon 34199 (KR)**
- **PARK, Seong Min**
  **Seoul 02105 (KR)**
- **JUNG, Jae Heum**
  **Busan 48050 (KR)**

(74) Representative: **Gevers Patents**
**Intellectual Property House**
**Holidaystraat 5**
**1831 Diegem (BE)**


(54) **METHOD FOR HYDROGENATION OF PHTHALATE COMPOUND**


(57) The invention relates to a method for hydrogenation of a phthalate compound. According to the method, the generation of by-products may be inhibited during hydrogenation, and thus, catalytic activity may be improved and catalyst life may be prolonged, thereby increasing the efficiency and economical feasibility of commercial processes. And, since the hydrogenation product prepared by the method has high purity and low acid value, it has excellent quality as a plasticizer, and thus, can be used for various products.


【FIG. 1】

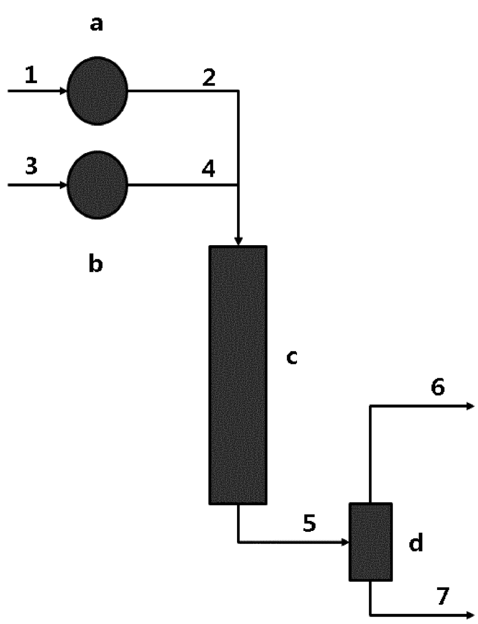


EP 3 719 000 A1

## Description

[Technical Field]

Cross-reference to Related Application

This application claims the benefit of Korean Patent Application No. 10-2017-0161951 filed on November 29, 2017 with the Korean Intellectual Property Office, the disclosures of which are herein incorporated by reference in their entirety.

The invention relates to a method for the hydrogenation of a phthalate compound. Specifically, it relates to a method for the hydrogenation of a phthalate compound that not only inhibits the generation of by-products and prolongs catalyst life, but also maintains the acid value of the hydrogenation product low, thereby improving the quality as a plasticizer.

[Background Art]

A phthalate based compound is material widely used as a plasticizer of plastic, particularly polyvinylchloride(PVC). For example, it is used for various applications such as electrical and electronic products, medicine, paint, lubricant, binder, surfactant, adhesive, tile, food containers, packaging material, and the like.

However, several phthalate compounds are known to cause environmental pollution and endocrine disruption of human, and the regulation of utilization is being strengthened around advanced countries such as Europe, the US, and the like. Particularly, among the phthalate-based plasticizers, some products such as di(2-ethylhexyl) phthalate (DEHP), butyl benzyl phthalate (BBP), or di-n-butyl phthalate (DBP) are suspected as endocrine disruptors that disturb or confuse the hormone action of human, and thus, there is a move for the regulation of the same.

Thus, efforts to develop environmentally friendly plasticizers that exhibit equivalent performance to the conventional plasticizers but are free from environmental hormone problem are being made, and one of them is a method of using a compound in which a benzene ring included in a phthalate compound is hydrogenated.

As the hydrogenation of an aromatic compound such as a benzene ring, a method of using a catalyst including a transition metal such as ruthenium as an active ingredient on a carrier, is known. For example, Korean Registered Patent No. 1556340 suggests a hydrogenation method of reacting a phthalate compound with hydrogen in the presence of a hydrogen catalyst and alcohol, and discloses that catalyst performance and life are improved according to this method.

The product prepared by the hydrogenation is obtained as a mixture of cis and trans isomers. Herein, as the content of cis isomers is higher, excellent plasticization efficiency for PVC resin, rapid absorption speed, and high product transparency after solidification are exhibited, and leaching on the product surface is less generated even after use for a long time, thus exhibiting excellent properties as a plasticizer.

Thus, in order to obtain a plasticizer having excellent quality, a hydrogenation method of a phthalate compound with improved stereoselectivity such that the content of cis isomers is high is required.

[Prior Art Technical Document]

Patent Document 1: Korean Registered Patent No. 1556340, "A Method of Hydrogenation of Phthalate Compound"

[Disclosure]

[Technical Problem]

In order to solve the above problem, it is an object of the invention to provide a novel method for the hydrogenation of a phthalate compound that not only inhibits side reactions and controls the acid value of the product, but also increases the purity of the product and improves the hydrogenation performance of a phthalate-based compound, thereby increasing reaction yield.

[Technical Solution]

In order to achieve the object, there is provided a method for the hydrogenation of a phthalate compound including the step of reacting a phthalate compound with hydrogen in the presence of a hydrogenation catalyst in a reactor, wherein a temperature deviation in the reactor is 0 to 30 °C during the reaction, and
the acid value of the hydrogenation product separated after the reaction is 0.3 KOHmg/g or less.

Preferably, during the reaction, a temperature change per unit length(m) of the reactor may be 10 °C or less.
The amount of hydrogen introduced into the reactor may be 3 to 300 moles, per 1 mole of the phthalate compound.
The phthalate compound may be one or more selected from the group consisting of phthalate, terephthalate, isophthalate and carboxylic acid compounds thereof.
The gas phase raw material may be fed from the upper part or lower part of the reactor, and the liquid raw material may be fed from the upper part of the reactor.
The hydrogenation catalyst may be one or more selected from the group consisting of ruthenium(Ru), rhodium(Rh), palladium(Pd) and platinum(Pt).
The amount of the hydrogenation catalyst may be 3 wt % or less, based on 100 wt % of a carrier.
There is also provided a hydrogenated phthalate or terephthalate compound, prepared by the above method.
The hydrogenated phthalate or terephthalate compound may be used as a plasticizer.
There is also provided a resin composition including the plasticizer, and a resin selected from ethylene vinyl acetate, polyethylene, polypropylene, polyvinyl chloride, polystyrene, polyurethane, polybutadiene, silicon, thermoplastic elastomer or copolymers thereof.

[Effect of the Invention]

According to the method for the hydrogenation of a phthalate compound, the generation of by-products may be inhibited during the hydrogenation reaction, and thus, catalytic activity may be improved and catalyst life may be prolonged, thereby increasing the efficiency and economical feasibility of commercial processes. And, since the hydrogenation product prepared by the method has high purity and low acid value, it has excellent quality as a plasticizer, and thus, can be used for various products.

[Brief Description of Drawings]

FIG. 1 briefly shows the hydrogenation apparatus used for the hydrogenation method of the invention.

[Best Mode]

Although various modifications can be made to the present invention and the present invention may have various forms, specific examples will be illustrated and explained in detail below. However, it should be understood that these are not intended to limit the present invention to a specific disclosure, and that the present invention includes all modifications, equivalents, or replacements thereof without departing from the spirit and technical scope of the invention.
As used herein, terms including an ordinal such as "first", "second" and the like are used to explain various constructional elements, but the constructional elements are not limited by the terms. The terms are used only to distinguish one constructional element from other constructional elements. For example, a first constructional element may be called a second constructional element, and similarly, a second constructional element may be called a first constructional element.
A singular expression includes a plural expression thereof, unless it is expressly stated or obvious from the context that such is not intended. As used herein, the terms "comprise", "have", etc. are intended to designate the existence of practiced characteristics, numbers, steps, constructional elements, or combinations thereof, and they are not intended to preclude the possibility of existence or addition of one or more other characteristics, numbers, steps, constructional elements, or combinations thereof.
Hereinafter, a method for the hydrogenation of a phthalate compound of the invention will be explained in detail with reference to drawings.
According to one preferable embodiment of the invention, there is provided a method for the hydrogenation of a phthalate compound including the step of reacting a phthalate compound with hydrogen in the presence of a hydrogenation catalyst in a reactor,
wherein a temperature deviation in the reactor is 0 to 30 °C during the reaction, and
the acid value of the hydrogenation product separated after the reaction is 0.3 KOHmg/g or less.
By minimizing a temperature deviation of the reactor during the reaction and blocking the generation of hot-spot, reaction efficiency may be increased and the generation of by-products may be inhibited. Thus, hydrogenation catalyst life in the reactor may be improved, and the effects of productivity improvement, yield increase, economical efficiency improvement may be secured. And, according to the hydrogenation method of the invention, the product may be prepared with an acid value below a certain level, thereby improving the quality as a plasticizer.
The hydrogenation subject is a phthalate compound, and by hydrogenation, hydrogen is added to the benzene ring of the phthalate compound, and the phthalate compound is converted into corresponding cyclohexane dicarboxylate.

The phthalate compound may be one or more selected from phthalate, terephthalate, isophthalate and corresponding carboxylic acid.
First, the phthalate compound may be represented by the following Chemical Formula 1.

[Chemical Formula 1]

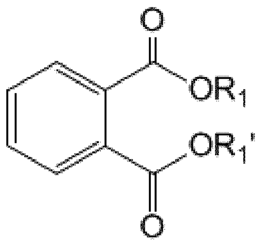

In the Chemical Formula 1, R1 and R1' are identical to or different from each other, and each independently, hydrogen, or a C1 to 20, preferably C4 to 20, more preferably C5 to 20, even more preferably C5 to 10 linear or branched alkyl group.
Specific examples of the phthalate compound may include dibutyl phthalate(DBP), dihexyl phthalate(DHP), dioctyl phthalate(DOP), di-n-octyl phthalate(DnOP), diisononyl phthalate, or diisodecyl phthalate(DIDP), but not limited thereto. These compounds may be used alone or in combinations.
The terephthalate compound may be represented by the following Chemical Formula 2.

[Chemical Formula 2]

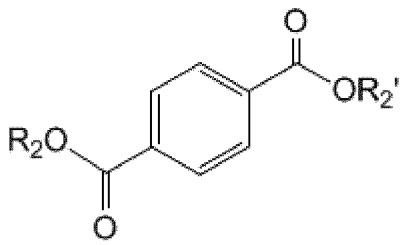

In the Chemical Formula 2, R2 and R2' are identical to or different from each other, and each independently, hydrogen, or a C1 to 20, preferably C4 to 20, more preferably C5 to 20, even more preferably C5 to 10 linear or branched alkyl group.
Specific examples of the terephthalate compound may include dibutyl terephthalate(DBTP), dioctyl terephthalate(DOTP), diisononyl terephthalate(DINTP), or diisodecyl terephthalate(DIDTP), but not limited thereto. These compounds may be used alone or in combinations.
The isophthalate compound may be represented by the following Chemical Formula 3.

[Chemical Formula 3]

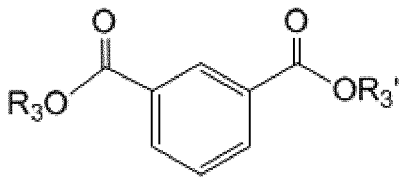

In the Chemical Formula 3, R3 and R3' are identical to or different from each other, and each independently, hydrogen, or a C1 to 20, preferably C4 to 20, more preferably C5 to 20, even more preferably C5 to 10 linear or branched alkyl group.
Specific examples of the isophthalate compound may include dibutyl isophthalalate(DBIP), dioctyl isophthalate(DOIP), diisononyl isophthalate(DINIP), or diisodecyl isophthalate(DIDIP), but not limited thereto. These compounds may be used alone or in combinations.

Preferably, as the phthalate compound, dioctyl terephthalate(DOTP) may be used.

The purity of the phthalate compound may be about 99 % or more, preferably about 99.5 % or more, more preferably about 98 % or more, but not limited thereto, and commercially available phthalate compounds of any qualities and purities may be used.

The hydrogenation process of the phthalate compound may be conducted in a liquid phase or gas phase. According to one embodiment of the invention, the hydrogenation process may be progressed with the phthalate compound in a liquid state, and hydrogen in a gas state.

The mass flux of the phthalate compound introduced into the reactor per unit area($m^2$), namely, the mass flux per unit area($m^2$) of the reactor filled with a catalyst may be preferably 10,000 to 100,000 $kg*hr^{-1}*m^{-2}$, more preferably 17,000 to 50,000 $kg*hr^{-1}*m^{-2}$.

If the mass flux of the phthalate compound per unit area($m^2$) is less than 10,000 $kg*hr^{-1}*m^{-2}$, input amount of raw material may not be sufficient, thus generating decrease in productivity, and if it is greater than 100,000 $kg*hr^{-1}*m^{-2}$, the amount of liquid raw material introduced in the reactor at once may excessively increase, and the thickness of a raw material film on the catalyst surface may increase, and thus, it may be difficult for hydrogen to penetrate, and it may be difficult for a hydrogenation reaction to occur, and thus, side reactions may increase and local heating may occur, thus increasing a temperature deviation of the reactor.

Meanwhile, in order to minimize side reactions and control the acid value of the hydrogenation product below a certain level, and optimize the ratio between reaction materials to improve productivity, the amount of hydrogen introduced in the reactor may be 3 moles or more, or 4 moles or more, or 7 moles or more, and 300 moles or less, or 100 moles or less, or 50 moles or less, or 30 moles or less, based on 1 mole of the phthalate compound.

If the amount of hydrogen is less than 3 moles per 1 mole of the phthalate compound, reaction conversion may be lowered, and conversion of 95 % or more may not be obtained, and if it is greater than 300 moles, the residence time of liquid drops of the liquid raw material may be shortened by hydrogen, and thus, conversion may decrease or by-products may increase, or catalyst life may be rapidly shortened. In this regard, it is preferable that the amount of hydrogen is within the above range.

The temperature and pressure conditions of the gas phase raw material and liquid phase raw material introduced in the reactor are not specifically limited, but the gas phase raw material may be controlled to a pressure of about 100 to about 200 bar, preferably about 130 to about 160 bar and a temperature of about 100 to about 200 °C, preferably about 130 to about 180 °C, and the liquid phase raw material may be controlled to a pressure of about 100 to about 200 bar, preferably about 130 to about 160 bar and a temperature of about 100 to about 200 °C, preferably about 130 to about 180 °C.

The hydrogenation catalyst may include transition metal as an active ingredient, and preferably, may include one or more selected from the group consisting of ruthenium(Ru), rhodium(Rh), palladium(Pd) and platinum(Pt).

Such a hydrogenation catalyst may be supported on a carrier, wherein any carriers known in the art may be used without limitations. Specifically, carriers such as zirconia($ZrO_2$), titania($TiO_2$), alumina($Al_2O_3$), silica($SiO_2$), and the like may be used.

In case the hydrogenation catalyst is supported on a carrier, the amount of the active ingredient of the hydrogenation catalyst may be preferably 3 wt % or less, 2 wt % or less, or 1 wt % or less, and 0.1 wt % or more, or 0.3 wt % or more, based on 100 wt % of the carrier. If the amount of the hydrogenation catalyst is greater than 3 wt % based on 100wt % of the carrier, reactions may be rapidly progressed on the catalyst surface, and during the process, side reactions may also increase and by-products may rapidly increase, and if it is less than 0.1 wt %, due to insufficient catalyst amount, the yield of a hydrogenation reaction may be lowered, and thus, the above range is preferable.

The hydrogenation reaction conditions are not specifically limited, but for example, the reaction pressure may be 50 bar or more, or 100 bar or more, or 130 bar or more, and 220 bar or less, or 200 bar or less, or 180 bar or less. If the reaction pressure is less than 50 bar, a reaction may not easily occur, and thus, excessive amount of catalyst may be consumed, residence time may excessively lengthen, thus increasing by-products and acid value, and if it is greater than 200 bar, excessive energy may be required during the operation of the process, and the manufacturing cost of equipment such as a reactor may significantly increase, and thus, the above range is preferable.

And, the reaction temperature may be 100 °C or more, or 120 °C or more, or 130 °C or more, and 300 °C or less, or 250 °C or less, or 200 °C or less. If the reaction temperature is less than 100 °C, reaction speed may be too slow, and thus, the reaction may not smoothly occur, and if it is greater than 300 °C, by-products may rapidly increase, thus significantly increasing the acid value of the product. And, it may also have an influence on the catalyst life, and thus, the above range is preferable.

By such a hydrogenation reaction, the aromatic ring of the phthalate compound is hydrogenated, and the phthalate compound is converted into corresponding cyclohexane dicarboxylate compound.

The reactor is controlled such that a temperature deviation in the reactor may be maintained 0 to 30 °C,

preferably 0 to 20 °C, more preferably 0 to 10 °C during the hydrogen reaction, thereby minimizing side reactions and improving reaction efficiency. Wherein, a temperature deviation means a difference between the maximum temperature and the minimum temperature in the reactor, and it may be measured by many temperature sensors installed according to the height of the reactor.

Alternatively, the reactor is controlled such that a temperature deviation per unit length(m) of the reactor is maintained 10 °C or less, or 5 °C or less during the hydrogen reaction, thereby avoiding local heating. Thus, side reactions other than hydrogenation may be inhibited, and the production amount of acidic by-products may be reduced, and thus, the hydrogenation product may have low acid value, and high purity and high quality product may be obtained. The lower the temperature deviation per unit length of the reactor, the better, and thus, the lower limit is not limited, but for example, the low limit may be 3 °C or more, preferably 0 °C. Such a temperature deviation may be measured from many temperature sensors installed according to the height of the reactor.

And, according to one embodiment of the invention, the maximum reaction amount per the actual reaction volume($m^3$) of the reactor filled with catalysts is maintained at 100 kmol/h or less, or 50 kmol/h or less, preferably 30 kmol/h or less during the hydrogenation reaction, so as not to generate local heating in the reactor, thereby inhibiting side reactions. Wherein, the reaction amount may be calculated through the concentration change value by respectively sampling the products according to the height of the reactor.

In order to control the reaction amount in the reactor, the mass flux or concentration of the reactant may be controlled as explained above, or for this purpose, inert gas or inert liquid may be added together with the reactant, and the amount may be controlled.

A method for controlling a temperature deviation and reaction amount in the reactor is not specifically limited, and any methods known in the art may be used. For example, a method of installing a cooling member in which a coolant is circulated, inside or outside of the reactor, to diffuse reaction heat may be used.

Wherein, as the coolant, any coolants known in the art, such as cooling water, a methane based coolant, a mixed coolant of fluorine-substituted or unsubstituted C1-5 lower alkane and ether, and the like, may be used without limitations. The kind of the cooling member is not specifically limited, but an indirect cooling method such as a heat exchanger, a cooling jacket, and the like, and a direct cooling method such as inert gas, inert liquid, and the like may be used.

In case the reaction is conducted while controlling a temperature deviation inside of the reactor, a reaction may uniformly occur inside of the reactor, and catalysts at the upper/lower part of the reactor may be uniformly loaded, thus significantly improving catalyst life. And, increase in side reactions due to the generation of hot spot may be significantly improved, thus obtaining a hydrogenation product having low acid value of 0.3 KOHmg/g or less, and high purity.

After the reaction is completed, a liquid phase hydrogenation product, and non-reacted gas phase raw material are separated. The separated gas phase raw material may be recycled to the hydrogenation process. And, the recovered hydrogenation product may be subjected to pressure reduction and cooling processes and finally separated.

According to the hydrogenation method of the invention, side reactions other than hydrogenation may be minimized, the production amount of acid by-products may be reduced, and thus, the hydrogenation product may exhibit low acid value, thus obtaining high purity and high quality product.

FIG. 1 is a drawing illustrating the hydrogenation apparatus used in the hydrogenation method of the invention.

Referring to FIG.1, the hydrogenation apparatus may consist of heat exchangers (a, b), a reactor (c) and a gas phase-liquid phase separator(d), and the like.

The heat exchangers (a, b) function for raising a temperature before introducing gas phase raw material (1) and liquid phase raw material (3) in the reactor (c), and may be omitted according to the necessity.

The gas phase raw material (2) and the liquid phase raw material (4) are introduced in a tubular type reactor (c) filled with a hydrogenation catalyst inside, thus progressing hydrogenation. The reactor may further include an external jacket for removing heat, so as to remove reaction heat. Wherein, the gas phase raw material (2) may be fed from the upper part or lower part of the reactor, and the liquid phase raw material (4) may be fed from the upper part of the reactor.

The reaction mixture (5) discharged from the reactor (c) is transferred to a gas phase-liquid phase separator (d), in which the liquid phase reaction product (7) and gas phase non-reacted material (6) are separated. The separated reaction product (7) may be recovered and subjected to additional purification process, and the gas phase non-reacted material (6) may be recycled for discharge or reuse.

However, in FIG. 1, the position of each piece of equipment may be changed, and if necessary, other equipment not shown in FIG. 1 may be included, and thus, the hydrogenation method of the invention is not limited to the equipment and process sequence shown in FIG. 1.

According to the hydrogenation method of the invention, side reactions may be inhibited, and thus, the acid value of the hydrogenation product may be controlled low, and catalytic activity may be improved and catalyst life may be prolonged, thereby improving the quality of the product and increasing the economical feasibility of commercial process.

The hydrogenated phthalate or terephthalate compound thus prepared may be usefully used as a plasticizer.

Specifically, a plasticizer including the phthalate or terephthalate compound may be used for a stabilizer, paint, ink, a liquid blowing agent(Masterbatch type), adhesive, and the like.

The phthalate or terephthalate compound prepared according to the above method has excellent purity and low acid value, and thus, has excellent quality as a plasticizer. Thus, it may be appropriately used for a plasticizer of resin selected from ethylene vinyl acetate, polyethylene, polypropylene, polyvinyl chloride, polystyrene, polyurethane, polybutadiene, silicon, thermoplastic elastomer or copolymer thereof.

A resin composition including the phthalate or terephthalate compound prepared according to the above method as a plasticizer, and including the above resin may be used for various products. For example, it may be used for the preparation of a film for food packaging(for example, a wrap), an industrial film, a compound, a decosheet, a decotile, a soft sheet, a hard sheet, a wire, and a cable, wall paper, a foaming mat, artificial leather, flooring, a tarpaulin, gloves, a sealant, a gasket for a refrigerator, a hose, a medical instrument, a geogrid, a mesh tarpaulin, toys, stationery, an insulating tape, a coating for clothes, PVC labels used for clothes or stationery, a stopper liner, stoppers for industrial or other uses, an artificial bait, parts in electronic equipment (for example, a sleeve), an automobile interior material, an adhesive, a coating, and the like, but is not limited thereto.

Hereinafter, the actions and effects of the invention will be explained in more detail through specific examples. However, these examples are no more than illustrations, and the scope of the right of the invention is not determined thereby.

**<Example>**

**Example 1**

Dioctyl terephthalate(DOTP) having purity of 99 % was introduced in a reactor, and hydrogenation was conducted at a reaction pressure of 150 bar and a reaction temperature of 165 to 175 °C.

The mass flux of DOTP pre unit area of the reactor($m^2$) was 17,000 kg/hr, and hydrogen was introduced at the hydrogen/DOTP mole ratio of 10. And, the volume flow ratio of hydrogen and DOTP was 5:1, pressure at the introduction of hydrogen was 150 bar, and pressure at the introduction of DOTP was 150 bar.

The reactor was in the form of a single-lumen tube, the total length of a part filled with catalyst in the tube was 3.0 m, and cooling fluid(Therminol 55) was flowed to the external jacket of the reactor to control such that a temperature deviation of the reactor was maintained at 15 to 25 °C during the reaction.

The catalyst used in the reactor was a ruthenium(Ru) catalyst having ruthenium content of 0.5 wt %, based on 100 wt % of an alumina($Al_2O_3$) carrier, and a cylinder type catalyst having a diameter of 3 mm and a height of 3 mm was used.

**Example 2**

Hydrogenation was conducted by the same method as Example 1, except that the reaction temperature was maintained at 155 to 165 °C, and the temperature deviation of the reactor was maintained at 7 to 16 °C during the reaction.

**Example 3**

Hydrogenation was conducted by the same method as Example 1, except that the reaction temperature was maintained at 140 to 155 °C, and the temperature deviation of the reactor was maintained at 8 to 17 °C during the reaction.

Comparative Example **1**

Hydrogenation was conducted by the same method as Example 1, except that the temperature deviation of the reactor was maintained at 25 to 35 °C during the reaction.

Comparative Example 2

Hydrogenation was conducted by the same method as Example 1, except that the temperature deviation of the reactor was maintained at 35 to 40 °C during the reaction.

**<Experimental Example>**

For the hydrogenation reactions of Examples 1 to 3 and Comparative Examples 1 to 2, conversion, by-products content, acid value of the hydrogenation product, acid value of the hydrogenation product after heating, and catalyst life

were evaluated.

The analysis of the reaction products and by-products was conducted using GC device(Agilent 6890 and 7890B) commonly used for the analysis of organic material, a mixture of 0.25mL of liquid analysis sample and 1mL of acetone was introduced in the GC analysis device, and the analysis time was set up as total 34 minutes to conduct analysis.

The acid value of the hydrogenation product was calculated by the following Mathematical Formula 1, after titrating the hydrogenation product obtained by the separation of un-reacted gas phase raw material from the reaction mixture with KOH.

[Mathematical Formula 1]

$$\text{Acid value} = \frac{0.561 \times \alpha \times \beta}{\delta}$$

$\alpha$: titration reagent(KOH) consumption amount, $\beta$: 1.00, $\delta$: amount of introduced sample(hydrogenation product)

The acid value after heating was obtained by maintaining the hydrogenation product separated from the reaction mixture at 125 °C for 3 hours, and then, titrating and calculating by the same method as above.

The catalyst life was calculated on the basis of conversion(xo) at the beginning of the reaction and conversion(Xi) 5 days after operation.

The evaluation results were summarized in the following Table 5.

[Table 1]

| Experimental Example | | Examp le 1 | Examp le 2 | Examp le 3 | Comparative Example 1 | Comparative Example 2 |
|---|---|---|---|---|---|---|
| Temperature deviation of reactor | °C | 15-25 | 7-16 | 8-17 | 25-35 | 35-40 |
| Catalyst | | Ru 0.5 | Ru 0.5 | Ru 0.5 | Ru 0.5 | Ru 0.5 |
| Conversion | % | 99.5 | 97.5 | 93.0 | 85.0 | 80.0 |
| Byproducts | wt % | 0.280 | 0.300 | 0.380 | 0.630 | 0.870 |
| Acid value | KOH mg/g | 0.015 | 0.130 | 0.270 | 0.351 | 0.520 |
| Acid value after heating | KOH mg/g | 0.120 | 0.164 | 0.291 | 0.514 | 0.795 |
| Catalyst life | $(X_0-X_1)/X_0$ | 0.99 | 0.98 | 0.96 | 0.75 | 0.55 |

Referring to Table 1, it is confirmed that in case a temperature deviation of the reactor is 30 °C or less, conversion and catalyst life are excellent, and by-products content in the product and acid value are low.

However, it is confirmed that in the case of Comparative Examples 1 and 2 wherein a temperature deviation of the reactor is greater than 30 °C, side reactions rapidly increase and by-products content significantly increases, and conversion and catalyst life remarkably decrease.

From the above experimental results, it is confirmed that a temperature deviation in the reactor during the hydrogenation of a phthalate compound has influence on reaction efficiency and the quality of the product, and it can be seen that according to the method of the invention, the quality of the product may be improved, and the efficiency and economical feasibility of commercial process may be maximized.

[Reference numerals]

a, b: heat exchangers
c: reactor

d: gas phase-liquid phase separator
1, 2: gas phase raw material
3, 4: liquid phase raw material
5: reaction mixture
6: gas phase un-reacted material
7: liquid phase reaction product

## Claims

1. A method for the hydrogenation of a phthalate compound comprising the step of reacting a phthalate compound with hydrogen in the presence of a hydrogenation catalyst in a reactor,
wherein a temperature deviation in the reactor is 0 to 30 °C during the reaction, and
the acid value of the hydrogenation product separated after the reaction is 0.3 KOHmg/g or less.

2. The method for the hydrogenation of a phthalate compound according to claim 1, wherein during the reaction, a temperature change per unit length(m) of the reactor is 10 °C or less.

3. The method for the hydrogenation of a phthalate compound according to claim 1, wherein the amount of hydrogen introduced into the reactor is 3 to 300 moles, per 1 mole of the phthalate compound.

4. The method for the hydrogenation of a phthalate compound according to claim 1, wherein the phthalate compound is one or more selected from the group consisting of phthalate, terephthalate, isophthalate and carboxylic acid compounds thereof.

5. The method for the hydrogenation of a phthalate compound according to claim 1, wherein gas phase raw material is fed from the upper part or lower part of the reactor, and liquid raw material is fed from the upper part of the reactor.

6. The method for the hydrogenation of a phthalate compound according to claim 1, wherein the hydrogenation catalyst is one or more selected from the group consisting of ruthenium(Ru), rhodium(Rh), palladium(Pd) and platinum(Pt).

7. The method for the hydrogenation of a phthalate compound according to claim 1, wherein the amount of the hydrogenation catalyst is 3 wt % or less, based on 100 wt % of a carrier.

8. A hydrogenated phthalate or terephthalate compound, prepared by the method according to any one of claims 1 to 7.

9. A plasticizer comprising the hydrogenated phthalate or terephthalate compound of claim 8.

10. A resin composition comprising the plasticizer of claim 9; and a resin selected from ethylene vinyl acetate, polyethylene, polypropylene, polyvinyl chloride, polystyrene, polyurethane, polybutadiene, silicon, thermoplastic elastomer or copolymer thereof.

【FIG. 1】

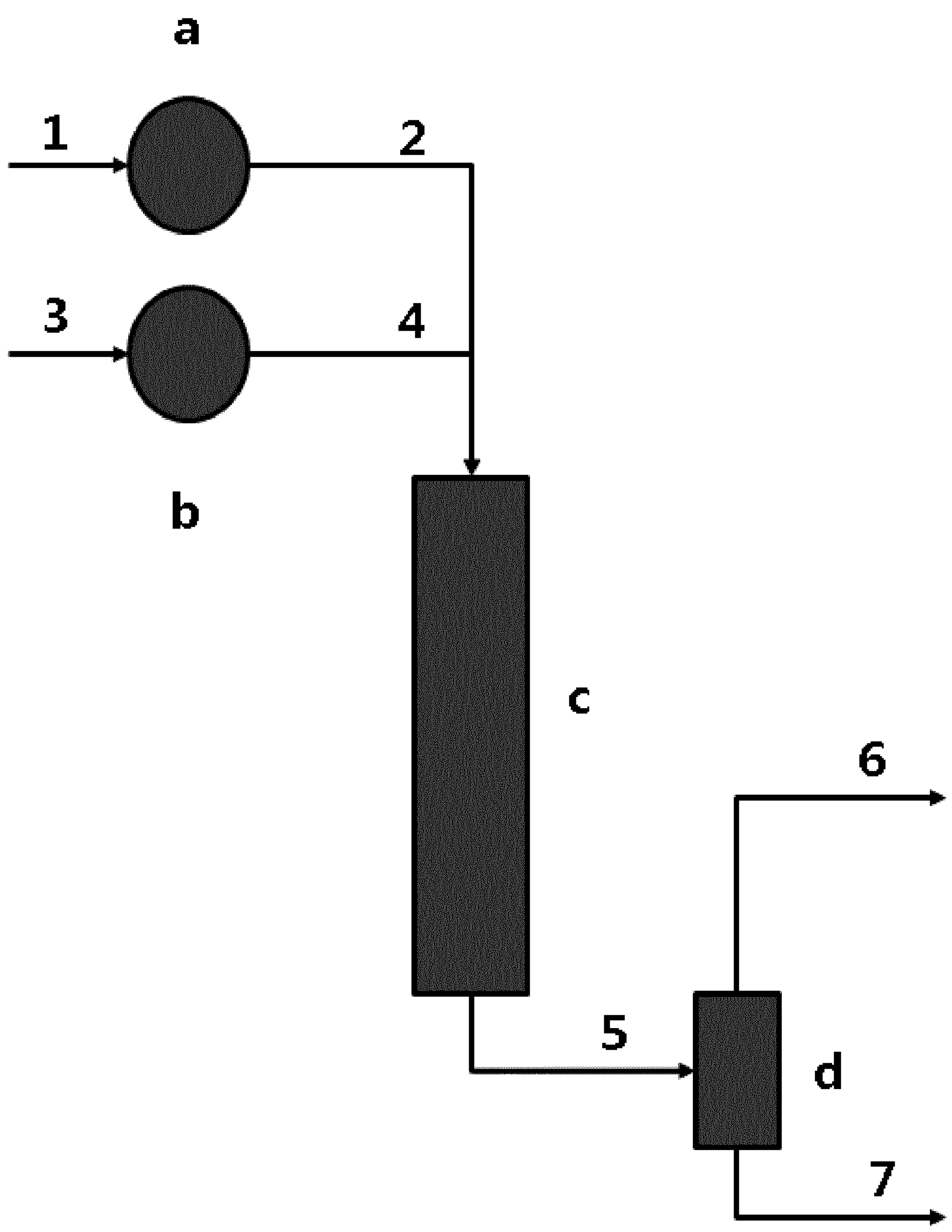

**INTERNATIONAL SEARCH REPORT**

International application No.
**PCT/KR2018/013196**

A. CLASSIFICATION OF SUBJECT MATTER
*C07C 67/303(2006.01)i, C07C 69/75(2006.01)i, B01J 23/46(2006.01)i, C08K 5/00(2006.01)i, C08K 5/101(2006.01)i, C08L 101/00(2006.01)i*
According to International Patent Classification (IPC) or to both national classification and IPC

B. FIELDS SEARCHED

Minimum documentation searched (classification system followed by classification symbols)
C07C 67/303; B01J 23/40; B01J 23/46; C07C 29/60; C07C 31/20; C07C 5/10; C07C 51/36; C07C 67/62; C07C 69/75; C08K 5/00; C08K 5/101; C08L 101/00

Documentation searched other than minimum documentation to the extent that such documents are included in the fields searched
Korean Utility models and applications for Utility models: IPC as above
Japanese Utility models and applications for Utility models: IPC as above

Electronic data base consulted during the international search (name of data base and, where practicable, search terms used)
eKOMPASS (KIPO internal) & Keywords: phthalate, hydrogenation, hydrogenation catalyst, temperature departure, acid value, by-product

C. DOCUMENTS CONSIDERED TO BE RELEVANT

| Category* | Citation of document, with indication, where appropriate, of the relevant passages | Relevant to claim No. |
|---|---|---|
| X<br>Y | KR 10-1797220 B1 (HANWHA CHEMICAL CORPORATION) 13 November 2017<br>See paragraphs [0002], [0023], [0056]-[0057]; example 1; table 2; and claims 1, 6, 11. | 1,2,4-10<br>3 |
| Y | KR 10-1550765 B1 (HANWHA CHEMICAL CORPORATION) 18 September 2015<br>See example 1. | 3 |
| A | KR 10-2016-0143620 A (HANWHA CHEMICAL CORPORATION) 14 December 2016<br>See the entire document. | 1-10 |
| A | KR 10-1657198 B1 (DOW GLOBAL TECHNOLOGIES INC.) 19 September 2016<br>See the entire document. | 1-10 |
| A | KR 10-0787690 B1 (CATALYTIC DISTILLATION TECHNOLOGIES)<br>21 December 2007<br>See the entire document. | 1-10 |

☐ Further documents are listed in the continuation of Box C. ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 12 APRIL 2019 (12.04.2019) | **12 APRIL 2019 (12.04.2019)** |

| Name and mailing address of the ISA/KR | Authorized officer |
|---|---|
| Korean Intellectual Property Office<br>Government Complex Daejeon Building 4, 189, Cheongsa-ro, Seo-gu, Daejeon, 35208, Republic of Korea<br>Facsimile No. +82-42-481-8578 | Telephone No. |

Form PCT/ISA/210 (second sheet) (January 2015)

**INTERNATIONAL SEARCH REPORT**
Information on patent family members

International application No.
**PCT/KR2018/013196**

| Patent document cited in search report | Publication date | Patent family member | Publication date |
|---|---|---|---|
| KR 10-1797220 B1 | 13/11/2017 | KR 10-2017-0025052 A | 08/03/2017 |
| | | WO 2017-034160 A1 | 02/03/2017 |
| KR 10-1550765 B1 | 18/09/2015 | CN 105793228 A | 20/07/2016 |
| | | EP 3085686 A1 | 26/10/2016 |
| | | JP 2016-539106 A | 15/12/2016 |
| | | JP 6441919 B2 | 19/12/2018 |
| | | KR 10-1556340 B1 | 30/09/2015 |
| | | KR 10-2015-0072197 A | 29/06/2015 |
| | | TW 201538473 A | 16/10/2015 |
| | | TW I534133 B | 21/05/2016 |
| | | US 2016-0280629 A1 | 29/09/2016 |
| | | WO 2015-093849 A1 | 25/06/2015 |
| KR 10-2016-0143620 A | 14/12/2016 | NONE | |
| KR 10-1657198 B1 | 19/09/2016 | CN 101970389 A | 09/02/2011 |
| | | CN 101970389 B | 05/11/2014 |
| | | EP 2262752 A1 | 22/12/2010 |
| | | EP 2262752 B1 | 19/11/2014 |
| | | KR 10-2010-0133369 A | 21/12/2010 |
| | | US 2011-0060168 A1 | 10/03/2011 |
| | | US 8324434 B2 | 04/12/2012 |
| | | WO 2009-111352 A1 | 11/09/2009 |
| KR 10-0787690 B1 | 21/12/2007 | CN 1174943 C | 10/11/2004 |
| | | CN 1371346 A | 25/09/2002 |
| | | EP 1218323 A1 | 03/07/2002 |
| | | EP 1218323 B1 | 09/05/2007 |
| | | JP 2003-510299 A | 18/03/2003 |
| | | KR 10-2002-0039665 A | 27/05/2002 |
| | | US 6187980 B1 | 13/02/2001 |
| | | WO 01-23334 A1 | 05/04/2001 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- KR 1020170161951
- KR 1556340